# EUROPEAN PATENT SPECIFICATION

(11) **EP 2 320 961 B1**
(45) Date of publication and mention of the grant of the patent: **28.10.2015**
(21) Application number: 09790930.3
(22) Date of filing: 29.07.2009
(51) Int. Cl.: A61L 27/14, A61L 27/48, A61L 27/58, A61L 31/04, A61L 31/12, A61L 31/14, A61L 27/18, A61L 27/34, A61L 31/06, A61L 31/10

(54) **MEDICAL ARTICLES COMPRISING BIODEGRADABLE BLOCK COPOLYMERS**
MEDIZINISCHE ARTIKEL MIT BIOLOGISCH ABBAUBAREN BLOCKCOPOLYMEREN
ARTICLES MEDICAUX CONTENANT DES COPOLYMERES BLOCS BIODEGRADABLES

(30) Priority: 31.07.2008 US 85130 P
(43) Date of publication of application: 18.05.2011
(73) Proprietor: Boston Scientific Scimed, Inc., Maple Grove, MN 55311-1566 (US)
(72) Inventor: EDELMAN, Peter, G., Maple Grove MN 55311 (US)
(74) Representative: Peterreins Schley
(86) International application number: PCT/US2009/052097
(87) International publication number: WO 2010/014703

(56) References cited:
- WO-A2-2008/002479
- US-A1- 2008 063 685

## Description

### FIELD OF THE INVENTION

The present invention relates to medical articles and more particularly to medical articles that comprise biodegradable polymers.

### BACKGROUND OF THE INVENTION

Various medical articles, including various state of the art medical devices consist of or contain biodegradable polymers. A problem with many biodegradable polymers is that, upon implantation, they may break down into fragments. When implanted in the vasculature, such fragments can cause embolisms and/or lead to infarcts. This fragmentation occurs due to a well-known process known as bulk erosion in which strength loss occurs much more quickly than mass loss, leading to fragmentation.

Copolymers are an important class of polymers and have numerous commercial applications. Their unique properties have lead to their use in a wide range of medical products. As a specific example, various state of the art medical devices consist of a medical device substrate with a copolymer-containing coating that serves as a reservoir for one or more therapeutic agents. Specific examples include drug eluting coronary stents, commercially available from Boston Scientific Corp. (TAXUS), Johnson & Johnson (CYPHER) and others. These products are based on metallic expandable stents with copolymer-containing coatings that release anti-restenotic drugs in a profile effective to inhibit the smooth muscle proliferation that is associated with restenosis (vessel reclosure). The polymer carrier technology in the TAXUS drug-eluting stent consists of a thermoplastic elastomer, poly(styrene-b-isobutylene-b-styrene) (SIBS), that results in optimal properties for a drug-delivery stent coating. S. Ranade et al., "Physical 5 characterization of controlled release of paclitaxel from the TAXUS™ Express2™ drug-eluting stent," Journal of Biomedical Materials Research Part A, 71 A (2004) 625-634. Block copolymers such as SIBS tend to phase separate into a hard phase domain corresponding to the styrene blocks and a soft elastomeric phase domain corresponding to the isobutylene block. Polymers of this type are capable of demonstrating high strength and elastomeric properties, while at the same time being processable using techniques such as solvent- and/or melt-based processing techniques.

There is an ongoing need for novel medical products, including pharmaceutical compositions and medical articles such as implantable and insertable medical devices, among others, particularly medical products comprising biodegradable copolymers.

US 2008/0063685 A 1 describes an implantable medical device comprising: a structural element, wherein the structural element includes: a continuous phase comprising a first polymer of LPLG; a discrete phase within the continuous phase, wherein the discrete phase comprises a second polymer including rapidly eroding elastic discrete phase segments. The second polymer further includes anchor segments that have the same or substantially the same chemical make up as the first polymer of the continuous phase, and at least some of the anchor segments have partially or completely phase-separated from the discrete phase into the continuous phase.

### SUMMARY OF THE INVENTION

The present invention is directed to a medical article comprising a polymeric region that comprises a biodegradable block copolymer comprising a first biodegradable polymer block and a second biodegradable polymer block that differs from the first biodegradable polymer block, said polymeric region comprising a first phase domain formed from said first polymer block and a second phase domain formed from said second polymer block.
wherein at least one of the first and second phase domains is a discontinuous phase domain in the form of dispersed phase elements, substantially all of which have a length that is less than 1 micron, and
wherein said first biodegradable polymer block is a low Tg polymer block and said second biodegradable polymer block is a high Tg polymer block.

### BRIEF DESCRIPTION OF THE DRAWING

FIG. 1 schematically illustrates several morphologies that may be formed from block copolymers that phase-separate into two immiscible phases, such as, for example, AB diblock and ABA triblock copolymers, among others.
FIG. 2A is a schematic illustration of a medical article in accordance with the present invention.
FIG. 2B is a schematic illustration of a medical article in accordance with another embodiment of the present invention.

### DETAILED DESCRIPTION OF THE INVENTION

The present invention is directed to medial articles having bioerodible polymeric regions that contain biodegradable block copolymers. The biodegradable block copolymers contain at least one first biodegradable polymer block and least one second biodegradable polymer block that differs from the first biodegradable polymer block. The bioerodible polymeric regions comprise a first phase domain that is formed from the at least one first biodegradable polymer block and a second phase domain that is formed from the at least one second biodegradable polymer block. At least one of the first and second phase domains is a discontinuous phase domain in the form of dispersed phase elements, (e.g., spheres, spheroids, rods, etc.) all or substantially all (at least 99% by number) of which have a longest cross-sectional dimension (e.g., the diameter for a sphere, the length for a rod, etc.) that is less than 1 micron (e.g., less than 1 micron, less than 500 nm, less than 250 nm, less than 100 nm, less than 50 nm, etc.).

In some embodiments, one of the first and second phase domains.is a continuous phase domain and the other of the first and second phase domains is a discontinuous phase domain whose dispersed phase elements are in the form spheres or other low-aspect ratio particles (i.e., those having an aspect ratio of 5 or less) having a longest cross-sectional length of 1 micron or less.

Dispersed phase elements of discontinuous phase domains can be visualized by various techniques known in the block copolymer art, including, for example, AFM (atomic force microscopy), TEM (transition electron microscopy) or SEM (scanning electron microscopy), after staining with suitable stain if desired.

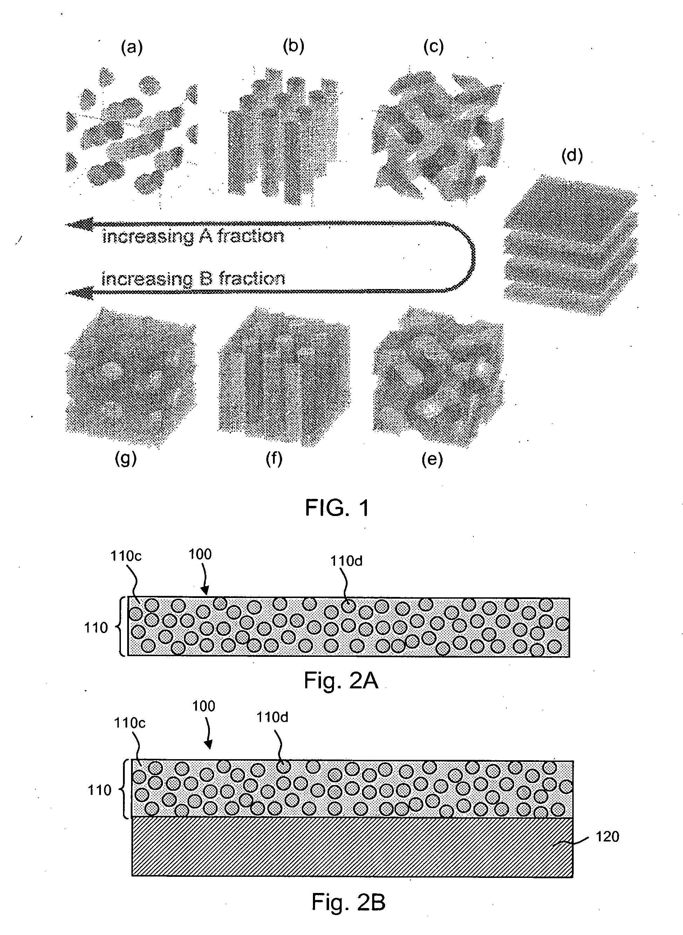

FIG. 2A is a schematic illustration of a medical article in accordance with an embodiment of the present invention, which consists of a bioerodible polymeric region 100 which contains a first continuous phase domain 110c and a second discontinuous phase domain 110d, which is in the form of dispersed phase elements (i.e., spheres) all or substantially all of which have a longest cross-sectional dimension (i.e., diameter) that is less than 1 micron.

FIG. 2A is a schematic illustration of a medical article in accordance with another embodiment of the present invention, which includes a substrate 120, upon which is disposed a bioerodible polymeric region 100 like that of FIG. 2A. Substrates 120 upon which polymeric regions in accordance with the invention 110 may be supported include biostable and bioerodible organic (e.g., polymeric) substrates and biostable and bioerodible inorganic (e.g. metallic or non-metallic) substrates.

As used herein, a "polymeric region" is a region (e.g., an entire article, a article component, a article coating layer, etc.) that contains polymers, for example, containing from 50 wt % or less to 75 wt % to 90 wt % to 95 wt % to 97.5 wt % to 99 wt % or more polymers.

As used herein, "polymers" are molecules containing multiple copies (e.g., 5 to 10 to 25 to 50 to 100 to 250 to 500 to 1000 or more copies) of one or more constitutional units, commonly referred to as monomers. As used herein, "monomers" may refer to free monomers and to those are incorporated into polymers, with the distinction being clear from the context in which the term is used.

Polymers may take on a number of configurations, which may be selected, for example, from linear, cyclic, and branched configurations. Branched configurations include star-shaped configurations (e.g., configurations in which three or more chains emanate from a single branch point), comb configurations (e.g., configurations having a main chain and a plurality of side chains), dendritic configurations (e.g., arborescent and hyperbranched polymers), network configurations (e.g., crosslinked polymers), and so forth.

As used herein, "homopolymers" are polymers that contain multiple copies of a single constitutional unit. "Copolymers" are polymers that contain multiple copies of at least two dissimilar constitutional units, examples of which include random, statistical, gradient, periodic (e.g., alternating) and block copolymers.

As used herein, "block copolymers" are copolymers that contain two or more polymer blocks that differ, for instance, because a constitutional unit (i.e., monomer) is found in one polymer block that is not found in another polymer block. As used herein, a "polymer block" is a grouping of constitutional units (e.g., 5 to 10 to 25 to 50 to 100 to 250 to 500 to 1000 or more units) that forms part or all of a polymer. Blocks can be branched or unbranched. Blocks can contain a single type of constitutional unit (also referred to herein as "homopolymer blocks") or multiple types of constitutional units (also referred to herein as "copolymer blocks") which may be provided, for example, in a periodic (e.g., alternating), random, statistical or gradient distribution.

Assuming they are of sufficient length, the differing polymer blocks of a given block copolymer in a given polymeric region are typically immiscible with one another and form distinct phases (or "phase domains") within the polymeric region. The shape; size, and spacing of the elements associated with each phase domain may be controlled by a number of factors including the processing techniques that are used to form the phase domains, the overall size (molecular weight) of the polymer blocks, and the size of the differing polymer blocks relative to one another (which affects the relative volume of each phase domain), among other factors.

FIG. 1 schematically illustrates morphologies that may be formed in a given polymeric region from a block copolymer that contains at least one polymer block A having a first monomer composition and at least one polymer block B having a different monomer composition (e.g., an AB diblock copolymer, an ABA triblock copolymer, etc.). The A blocks form a first phase domain represented by dark grey shading in FIG. 1, while the B blocks form a second phase domain represented by light grey/white shading in FIG. 1. As the relative volume occupied by phase A goes from high to low, for example, as the molecular weight of the B block(s) increase relative to that of the A blocks, morphologies that may be encountered include: (a) spheres of phase B (a discontinuous phase) in a matrix of phase A (a continuous phase), (b) cylinders of phase B (a discontinuous phase) in a matrix of phase A (a continuous phase), (c) dual labyrinths of phase B and in a matrix of phase A (e.g., double gyroid), otherwise known as a bi-continuous system, (d) alternating sheets of phase A and phase B (e.g., lamellae) (a "bi-discontinuous" system), (e) dual labyrinths of phase A in a matrix of phase B, (f) cylinders of phase A in a matrix of phase B, and (g) spheres of phase A in a matrix of phase B. More complex configurations may be exhibited by polymers having three distinct phases A, B and C.

As used herein, a polymer or a polymer block is "biodegradable" if it undergoes bond cleavage along the polymer backbone in vivo, regardless of the mechanism of bond cleavage (e.g., enzymatic breakdown, hydrolysis, oxidation, etc.).

As used herein, "bioerosion" is a result of biodegradation (as well as other in vivo disintegration processes such as dissolution, etc.) and is characterized by loss over time of the original mass of a polymeric region. Polymeric regions (a) may bioerode primarily from the surface inward over time (surface erosion), (b) may erode primarily throughout their bulk over time (bulk erosion), which as noted above, can lead to fragmentation of the polymeric region, or (c) may undergo a combination of bulk and surface erosion.

By way of background and without wishing to be bound by theory, polymer biodegradation can occur from a variety of bond cleavage mechanisms (e.g., hydrolysis, enzymatic breakdown, etc.), and generally requires exposure of the polymer to water (and in some instances other species such as catalysts). As noted above, a polymer can undergo surface erosion, bulk erosion or a combination of both. As a specific example, hydrolysis occurs in biodegradable polymers such as polyanhydrides, polyesters and polyorthoesters, among others, when water contacts the same. If hydrolysis proceeds quickly relative to the rate of water penetration into the polymer bulk, surface erosion will predominate. If hydrolysis proceeds slowly relative to the rate of water penetration into the polymer bulk, bulk erosion will predominate. Thus polymers having relatively high rates of hydrolysis relative to water penetration, such as polyanhydrides and polyorthoesters, are commonly referred to as surface eroding polymers, whereas polymers having relatively slow rates of hydrolysis relative to water penetration, such a polyesters, are commonly referred to as bulk eroding polymer.

According to the present invention, the bioerodible polymeric regions of the present invention comprise a biodegradable block copolymer that contains at least one low glass transition temperature (Tg) biodegradable polymer block and at least one high Tg biodegradable polymer block. The bioerodible polymeric regions comprise a first phase domain formed from the at least one low Tg biodegradable polymer block and a second phase domain formed from the at least one high Tg biodegradable polymer block.

As used herein, "low Tg polymer blocks" are those that display a Tg that is below body temperature (37° C. for humans), more typically 35° C. to 20° C. to 0° C. to -25° C. to -50° C. or below. Conversely, elevated or "high Tg polymer blocks" are those that display a glass transition temperature that is above body temperature, more typically 40° C. to 50° C. to 75° C. to 100° C. or above. Tg can be measured by any of a number of techniques including differential scanning calorimetry (DSC). In general, low Tg polymer blocks are soft and elastomeric at body temperature, whereas high Tg polymer blocks are hard.

In certain embodiments, block copolymers in accordance with the invention may contain at least two high Tg polymer blocks separated from one another by at least one low Tg block (in other words, block copolymers in which high Tg blocks are interconnected via a low Tg block).

Examples include, for example, the following configurations (in which "L" designates a linear low Tg polymer block and "H" designates a linear high Tg polymer block) among many others: (a) block copolymers having alternating blocks of the type HLH, (LH)m, L(HL)m and H(LH)m where m is a positive whole number of 2 or more, (b) multiarm (including star) copolymers such as X(LH)m, where X is a hub species (e.g., an initiator molecule residue, a linking residue, etc.) m is a positive whole number of 2 or more, and (c) comb copolymers having a low Tg polymer backbone and multiple high Tg side chains. Note that non-monomeric species are commonly ignored in block copolymer nomenclature. For example, in the copolymer X(LH)m where m=2 (i.e., HLXLH), the hub species is typically ignored, and the polymer is identified as an HLH triblock copolymer.

Block copolymers like those described in the foregoing paragraph tend to phase separate into a hard phase domain (formed from the H blocks) and an elastomeric phase domain (formed from the L blocks). Such polymers are capable of demonstrating good flexibility/elasticity and strength, while at the same time being processable using techniques such as solvent- and/or melt-based processing techniques.

Examples of polymer blocks that may be used to form the low and high Tg biodegradable polymer blocks of the copolymers of the invention include polyester, polyether-ester, polyorthoester, polyanhydride, polycarbonate, and polyamide-ester blocks. Several specific examples are given below, and Tg's are reported where available. Sources of Tg information include I. Engelberg et al., Biomaterials, 12 (1991) 292-304 and M. Zilberman et al., Annu. Rev. Biomed. Eng., 8 (2006) 153-80.

Biodegradable polymer blocks include polyester polymer blocks, for example, homopolymer and copolymer blocks that comprise alpha-hydroxyacids (e.g., glycolic acid, l-lactic acid, d-lactic acid, etc.), alpha- and beta-hydroxyalkanoic acids (e.g., 3-hydroxybutyrate, 3-hydroxyvalyrate, etc.), and lactones in addition to those associated with the foregoing hydroxyacids (e.g., epsilon-caprolactone). Specific examples include high Tg biodegradable polyester blocks such as poly(l-actide) (Tg 60-65° C.), poly(d,l-lactide) (Tg 55-60° C.), and poly(d,l-lactide-co-glycolide) (Tg 45-55° C.) blocks. Of these, poly(l-lactide) is semi-crystalline, which may provide additional strength to the polymeric region into which it is incorporated. Specific examples further include low Tg biodegradable polyester blocks such as poly(epsilon-caprolactone) (Tg -62° C.), poly(3-hydroxybutyrate) (Tg 1° C.), and poly(3-hydroxybutyrate-co-3-hydroxyvalyrate) blocks, which have reported Tg's of around 0° C. In certain embodiments, racemic mixtures may be preferred (e.g., poly(R,S-3-hydroxybutyrate)).

Biodegradable polymer blocks further include biodegradable polyether-ester polymer blocks, for example, homopolymer and copolymer blocks that comprise ether-ester monomers such as p-dioxanone, for example, poly(p-dioxanone) (Tg -10 to 0° C.), among others.

Biodegradable polymer blocks further include homopolymer and copolymer blocks that comprise iminocarbonates. A specific example of a polyiminocarbonate is poly(desaminotyrosine-tyrosoine-hexyl ester-iminocarbonate) (Tg 55° C.). For more information, see, e.g., I. Engelberg et al., Biomaterials, 12 (1991) 292-304 and the references cited therein.

Biodegradable polymer blocks further include polycarbonates, for example, homopolymer and copolymer blocks that comprise alkylene carbonates such as ethylene carbonate (1,3-dioxolan-2-one), propylene carbonate (4-methyl-1,3-dioxolan-2-one), trimethylene carbonate (1,3-dioxan-2-one), tetramethylene carbonate, and so forth. Specific examples include poly(ethylene carbonate) (Tg 10 to 30° C.) and poly(trimethylene carbonate) (Tg -15° C.).

Biodegradable polymer blocks also include polyanhydrides, for example, homopolymer and copolymer blocks that comprise one or more multivalent acids such as the following: sebacic acid (SA), bis-(p-carboxyphenoxylpropate) (CPP), isophthalic acid (ISO), hexadecandioic acid (HDA), fumaric acid (FA), terephthalic acid (TA), adipic acid (AA) and dodecanedioic acid (DD). Specific examples of high Tg polyanydride blocks include poly(CPP-ISO) (20:80) (Tg 110° C.), poly(CPP-ISO) (50:50) (Tg 100° C.), poly(CPP-ISO) (75:25) (Tg 230° C.), poly(CPP-ISO-SA) (15:58:27) (Tg 46° C.), poly(CPP-ISO-SA) (17:66:16) (Tg 83° C.), poly(CPP-ISO-TA) (50:40:10) (Tg 111.6° C.), and poly(CPP-ISO-TA) (25:60:15) (Tg 105° C.), among others. See U.S. Pat. No. 4,997,904 to Domb. Specific examples of low Tg polyanhydride blocks include poly(CPP-SA) (46:54) (Tg 1.6° C.), poly(EAD) (Tg <0° C.), poly(EAD-SA) (22:78) (Tg <10° C.) arid poly(EAD-SA) (8:92) (Tg <10° C.). See Polymer Data Handbook, James E. Mark, Ed., Oxford University Press, 1999, pp. 303-4 and 457-8.

Biodegradable polymer blocks further include polyorthoesters. Examples of polyorthoesters include those that are formed by the reaction between a diketene acetal, for example, 3,9-diethylidene-2,4,8,10-tetraoxaspiro[5,5]undecane (DETOSU), with a diol. The use of rigid diols gives high Tg polymers. For example, the use of trans-cyclohexanedimethanol (tCDM) as a diol produces a poly(DETOSU-tCDM) copolymer having a Tg 120° C. The use of flexible diols gives low Tg polymers. For example, the use of 1,6 hexane diol (1,6-HD) produces a poly(DETOSU-1,6-HD) copolymer having a Tg of 20° C. Higher diols (e.g., 1,8-octane diol, 1,10-decane diol, 1,12-dodecane diol, etc.) produce polymers whose Tg's decrease with increasing diol length. For example, 1,12-dodecane diol yields a copolymer having a Tg of approximately 0° C. Mixtures of rigid and flexible diols give intermediate Tg's. For example, copolymers of DETOSU, and a mixture of t-CDM and 1,6-HD have reported Tg's of 55° C. (t-CDM:1,6-HD=35:65), 84° C. (t-CDM:1,6-HD=70:30), 95° C. (t-CDM:1,6-HD=90:10). Poly(ortho esters) formed from higher diols are more hydrophobic than those formed from lower diols and, consequently, have slower degradation rates. Because the ortho ester linkages are acid labile, degradation can be increased by introducing acidic species and decreased by the introduction of alkaline species. Examples of basic species that have been employed for this purpose include Mg(OH)2. Examples of acidic species that have been employed include free acids and latent acids that are incorporated into the polymer structure. Specific examples include suberic acid and naltrexone palmoate. Further specific examples include latent acid diols (e.g., diols based on monomers, dimers, trimers, etc. of hydroxy acids such as lactic acid, glycolic acid, etc.), which can be incorporated into the polymer chain and which produce acidic species upon degradation. Such latent acid species are also known to affect Tg. For example, poly(ortho esters) prepared from DETOSU, lactic acid diol, and one of 1,8-octane diol, 1,10-decane diol and 1,12-dodecane diol, were all found to display lower Tg's with increasing amounts of latent acid diol. For further information on polyorthoesters see, e.g., Handbook of Biodegradable Polymers, Abraham J. Domb, Joseph Kost, David M. Wiseman, Eds., CRC Press, 1997, Chapter 6, Scaffolding in Tissue Engineering, Peter X. Ma and Jennifer Elisseeff, Eds., CRC Press, 2005, Chapter 7, and the references cited therein.

As previously noted, polyorthoesters and polyanhydrides generally erode by surface erosion.

In certain aspects, the bioerodible polymeric regions of the invention comprise a biodegradable block copolymer that contains at least one block formed from a surface erodible polymer (surface erodible polymer block) and at least one additional polymer block. The polymeric bioerodible regions comprise a continuous phase domain formed from the at least one surface erodible polymer block and a discontinuous phase domain formed from the at least one additional polymer block, whose dispersed phase elements (e.g., dispersed spheres, spheroids, rods, etc.) are all or substantially all less than 1 micron in largest cross-sectional length. The surface erodible blocks and additional polymer blocks are selected such that the continuous phase domain bioerodes at a faster rate than the discontinuous phase domain. Consequently, the continuous phase domain (because it is formed from surface erodible polymer blocks) bioerodes from the outer surface of the polymeric region inward, releasing the particles corresponding to the discontinuous phase domain as bioerosion progresses. As noted above, a problem with many biodegradable polymers is that, upon implantation into the vasculature, they may break down into fragments that cause embolisms and/or lead to infarcts. In accordance with the present aspect of the invention, degradation results in particles which are less than 1 micron. At this sub-micron size, there is no risk of embolism or infarct, particularly given that the particles are released in a controlled fashion.

In certain embodiments the at least one surface erodible polymer block is a low Tg polymer block and the at least one additional polymer block is a high Tg polymer block. In certain embodiments the at least one surface erodible polymer block is a high Tg polymer block and the at least one additional polymer block is a low Tg polymer block. For example, copolymers may be formed having one of the above architectures in which high Tg blocks are interconnected via a low Tg block, for instance, an HLH triblock copolymer, among numerous others.

Examples of low Tg surface erodible polymer blocks include low Tg polyanhydride blocks and low Tg polyorthoesters blocks. More specific examples of low Tg polyanhydride blocks include low Tg poly(EAD), poly(EAD-SA) and poly(CPP-SA) such as those described above. More specific examples of low Tg polyorthoesters blocks include those formed from DETOSU and a flexible diol, for example, a C6-C12 diol, optionally, with an acid species, such as a latent acid species, to accelerate degradation.

Examples of high Tg additional polymer blocks include high Tg polyester polymer blocks and high Tg polyiminocarbonate blocks. More specific examples include poly(l-lactide), poly(d,l-lactide), poly(d,l-lactide-co-glycolide), and poly(desaminotyrosine-tyrosoine-hexyl ester-iminocarbonate). Further examples of high Tg additional polymer blocks include high Tg polyarylates as disclosed in S. Brocchini et al., Journal of Biomedical Medical Research, 42(1), 66-75, (1998).

Examples of high Tg surface erodible polymer blocks include high Tg polyanhydride blocks and high Tg polyorthoesters blocks. More specific examples of high Tg polyanhydrides blocks include those formed from one or more of CPP, ISO, SA, TA, FA, AA, DD and HDA, which may be selected from those described above, among others. More specific examples of high Tg polyorthoesters include those formed from DETOSU and a rigid diol, for example, t-CDM, optionally, with an acid species, such as a latent acid species, to accelerate degradation.

Examples of low Tg additional polymer blocks include low Tg polyester blocks, low Tg polyether-ester blocks, and low Tg polyalkylene carbonate blocks. More specific examples include poly-epsilon-caprolactone, poly(3-hydroxybutyrate), poly(3-hydroxybutyrate-co-3-hydroxyvalerate), poly(p-dioxanone) and poly(trimethylene carbonate).

As previously indicated, the various biodegradable block copolymers described herein include at least one first biodegradable polymer block and least one second biodegradable polymer block that differs from the first biodegradable polymer block. The block copolymers form a first phase domain from the at least one first biodegradable polymer block and a second phase domain from the at least one second biodegradable polymer block.

In certain embodiments, the block copolymers of the invention are provided with weak linkages between the first and second polymer blocks such that the first and second phase domains become more readily decoupled from one another in vivo. Examples of such weak linkages include short sequences (e.g., ranging from 1 to 2 to 5 to 10 to 20 monomers) composed of monomers that form rapidly degrading linkages (i.e., having a higher degradation rate than the first and second polymer blocks). Specific examples of such sequences include glycolate sequences and succinate sequences. The first and second polymer blocks are typically much longer in length, for example, comprising block lengths such that the block molecular weight is on the order of 10,000 Daltons and greater in certain embodiments.

Block copolymers in accordance with the invention can be formed using various techniques known in the block copolymer art, including successive polymerization reactions, polymerizations that employ macroinitiators and macromonomers, and techniques wherein previously formed polymers are coupled to form block copolymers. For example, a difunctional initiator X can be used to perform a first polymerization of first biodegradable polymer chains (A), followed by a second polymerization of second biodegradable polymer chains (B), forming a block copolymer BAXAB which is represented as a triblock copolymer BAB, as noted above. As another example, a difunctional macroinitiator (e.g., one comprising a first biodegradable polymer chain (A) with initiator groups at each end) may be used to initiate polymerization of second biodegradable polymer chains (B) to yield a BAB triblock copolymer. As yet another example, two first biodegradable polymer chains (A), each having reactive functional groups at one end, may be coupled to a second biodegradable polymer chain (B) having reactive functional groups at both ends, thereby forming an ABA triblock copolymer.

As previously noted, bioerodible polymeric regions formed from the biodegradable block copolymers of the invention comprise at least one discontinuous phase domain, whose dispersed phase elements (e.g., dispersed spheres, rods, etc.) are all or substantially all less than 1 micron in largest cross-sectional length (e.g., sphere diameter, rod length, etc..).

The morphology (e.g., size, shape, spacing, etc.) of the phase domains that are produced by the block copolymers may be affected by a number of factors, including the nature of the block copolymer itself (e.g., the number and length of the various biodegradable polymer blocks within the copolymer) and the processing technique that is used to form the polymeric region.

For example, the greater the molecular weight of a first polymer block relative to that of a second polymer block, the greater the volume fraction of the phase corresponding to the first polymer block relative to the volume fraction of the phase corresponding to the second polymer block. By varying the volume fraction of the various phase domains in this fashion, different morphologies may be created (see, e.g., the spherical, cylindrical, lamellar and double gyroid morphologies of FIG. 1, discussed above, among other possibilities).

Note that, in some embodiments, the size of the dispersed phase elements associated with the at least one discontinuous phase domain may be reduced by reducing the overall size (e.g., the molecular weight) of the polymer blocks that form the discontinuous phase domain, up to the point where the polymer blocks are so small that they do not produce recognizably distinct phases, even under microscopy.

As a general rule of thumb, processing conditions approaching equilibrium conditions tend to produce more regular structures such as spheres and rods. Annealing processes may also be employed in conjunction with phase domain formation, particularly those approximating an equilibrium phase domain arrangement.

In certain embodiments, polymeric regions in accordance with the invention are provided with a therapeutic agent, for example, an anti-restenotic agent, an anti-proliferative agent, an anti-inflammatory agent or an antimicrobial agent, among many other possibilities. The therapeutic agent, for example, may preferentially associate with the first phase domain formed from the one or more first polymer blocks (e.g., a continuous phase domain, etc.), may preferentially associate with the second phase domain formed from the one or more second polymer blocks (e.g., a discontinuous phase domain, etc.), or may preferentially occupy an interface between the first and second phase domains.

In certain embodiments, solvent-based techniques are used to form the polymeric regions of the present invention. Using these techniques, polymeric regions can be formed by first forming a solution that contains the biodegradable block copolymer(s) that will ultimately provide the phase domains, followed by removal of the solvent, which leads to phase separation and the creation of the phase domains. The solvent selected will contain one or more solvent species, and it is generally selected based on its ability to dissolve the copolymer(s) that form the polymeric region as well as other factors, including drying rate, surface tension, etc. Generally several solvents will be tested to see which provides polymeric regions having the best characteristics. Processing factors that may affect the morphology of the phase domains include the particular solvent system selected, the concentration of the copolymer(s) in the solvent system, the temperature at which solvent evaporation proceeds, the rate of evaporation of the solvent system, and so forth.

Solvent-based techniques include, for example, solvent casting techniques, spin coating techniques, web coating techniques, solvent spraying techniques, dipping techniques, coating techniques employing an applicator such as a brush, roller or sponge, ink jet techniques, and techniques involving coating via mechanical suspension including air suspension, among others.

A variety of medical articles, including various implantable or insertable medical devices, may be provided with polymeric regions in accordance with the invention, including, for example, stents (including vascular stents such as coronary stents, peripheral vascular stents, and cerebral stents, urethral stents, ureteral stents, biliary stents, tracheal stents, gastrointestinal stents and esophageal stents), stent coverings, stent grafts, vascular grafts, abdominal aortic aneurysm (AAA) devices (e.g., AAA stents, AAA grafts, etc.), vascular access ports, dialysis ports, catheters (e.g., urological catheters or vascular catheters such as balloon catheters and various central venous catheters), guide wires, balloons, filters (e.g., vena cava filters and mesh filters for distil protection devices), embolization devices including cerebral aneurysm filler coils (including Guglielmi detachable coils and metal coils), embolic agents, septal defect closure devices, drug depots that are adapted for placement in an artery for treatment of the portion of the artery distal to the device, myocardial plugs, patches, pacemakers, leads including pacemaker leads, defibrillation leads and coils, neurostimulation leads such as spinal cord stimulation leads, deep brain stimulation leads, peripheral nerve stimulation leads, cochlear implant leads and retinal implant leads, ventricular assist devices including left ventricular assist hearts and pumps, total artificial hearts, shunts, valves including heart valves and vascular valves, vascular closure devices (particularly the intra-arterial portions thereof), anastomosis clips and rings, cochlear implants, tissue bulking devices, tympanostomy tubes, thoracic drainage tubes, nephrostomy tubes, and tissue engineering scaffolds for cartilage, bone, skin, nerve (e.g., for neural pathway regeneration, including the spinal cord,), and other in vivo tissue regeneration, sutures, suture anchors, tissue staples and ligating clips at surgical sites, cannulae, metal wire ligatures, urethral slings, hernia "meshes", artificial ligaments, tacks for ligament attachment and meniscal repair, joint prostheses, spinal discs and nuclei, orthopedic prosthesis such as bone grafts, bone plates, fins and fusion devices, orthopedic fixation devices such as interference screws in the ankle, knee, and hand areas, rods and pins for fracture fixation, screws and plates for craniomaxillofacial repair, dental implants, contact lenses, intraocular lenses, punctum plugs, glaucoma shunts, or other devices that are implanted or inserted into the body.

As noted above, polymeric regions in accordance with the invention include those which correspond to an entire medical article or to only a portion of a medical article (e.g., to a polymeric coating layer, a distinct component of a medical device, etc.). For example, a polymeric coating layer in accordance with the invention may be provided over the entire surface of the medical article or over only a portion of a medical article surface.

In a specific example of a tubular medical devices such as a stent (which can comprise, for example, a laser cut or mechanically cut tube, one or more braided, woven, or knitted filaments, etc.), polymeric regions (e.g., coatings) in accordance with the invention may be provided over the entire surface of the stent, or they may be provided on the inner luminal surface of the stent, on the outer abluminal surface of the stent, and/or on the lateral surfaces between the luminal and abluminal surfaces (including the ends). The polymeric coatings may be provided in desired patterns, for instance, using appropriate masking techniques, among others.

To reduce or prevent polymer degradation, medical devices in accordance with the present invention may be packaged in an anhydrous state (e.g., packaged under vacuum, in a dry inert atmosphere, in an anhydrous solvent, etc.).

## Claims

1. A medical article comprising a polymeric region that comprises a biodegradable block copolymer comprising a first biodegradable polymer block and a second biodegradable polymer block that differs from the first biodegradable polymer block, said polymeric region comprising a first phase domain formed from said first polymer block and a second phase domain formed from said second polymer block,
wherein at least one of the first and second phase domains is a discontinuous phase domain in the form of dispersed phase elements, substantially all of which have a length that is less than 1 micron, and
wherein said first biodegradable polymer block is a low Tg polymer block and said second biodegradable polymer block is a high Tg polymer block.

2. The medical article of claim 1, wherein said medical article is an implantable or insertable medical device.

3. The medical article of claim 2, wherein said medical device is a vascular medical device selected from a stent, a graft, a stent graft, a vena cava filter, an embolic coil, a heart valve, a left ventricular access device, an artificial heart and a vascular closure device.

4. The medial article of claim 1, wherein said dispersed phase elements comprise spherical elements.

5. The medical article of claim 1, wherein said block copolymer is a triblock copolymer comprising a low Tg midblock and high Tg endblocks.

6. The medical article of claim 5, wherein said low and high Tg polymer blocks are biodegradable polyester blocks.

7. The medical article of claim 5, wherein said low Tg polymer block is selected from homopolymer and copolymer blocks that comprise a monomer selected from epsilon-caprolactone, 3-hydroxybutyrate, 3-hydroxyvalerate, and combinations thereof, and wherein said high Tg block is selected from homopolymer and copolymer blocks that comprise a monomer selected from l-lactide, d-lactide, glycolide and combinations thereof.

## Patentansprüche

1. Ein medizinischer Gegenstand, umfassend eine polymere Region, welche ein bioabbaubares Blockcopolymer, umfassend einen ersten bioabbaubaren Polymerblock und einen zweiten bioabbaubaren Polymerblock, der sich von dem ersten bioabbaubaren Polymerblock unterscheidet, umfasst, wobei die polymere Region eine erste Phasendomäne, die aus dem ersten bioabbaubaren Polymerblock gebildet ist, und eine zweite Phasendomäne, die aus dem zweiten bioabbaubaren Polymerblockgebildet ist, umfasst,
wobei mindestens eine der ersten und zweiten Phasendomänen eine nichtkontinuierliche Phasendomäne in Form von dispergierten Phasenelementen ist, die im Wesentlichen alle eine Länge von weniger als 1 Mikrometer aufweisen, und
wobei der erste bioabbaubare Polymerblock ein Polymerblock mit niedrigem Tg ist und der zweite bioabbaubare Polymerblock ein Polymerblock mit hohem Tg ist.

2. Der medizinische Gegenstand nach Anspruch 1, wobei der medizinische Gegenstand ein implantierbares oder einführbares medizinisches Gerät ist.

3. Der medizinische Gegenstand nach Anspruch 1, wobei der medizinische Gegenstand ein vaskulärer medizinischer Gegenstand ist, welcher unter einem Stent, ein Transplantat, ein Stenttransplantat, ein Vena-Cava-Filter, eine embolische Spirale, eine Herzklappe, eine linksventrikuläre Zuführvorrichtung, ein künstliches Herz und eine vaskuläre Verschlußvorrichtung gewählt ist.

4. Der medizinische Gegenstand nach Anspruch 1, wobei die dispergierten Phasenelemente kugelförmige Elemente umfassen.

5. Der medizinische Gegenstand nach Anspruch 1, wobei das Blockcopolymer ein Triblock-Copolymer, umfassend einen mittleren Block mit niedrigem Tg und Endblöcke mit hohem Tg, ist.

6. Der medizinische Gegenstand nach Anspruch 5, wobei die Polymerblöcke mit niedrigem und hohem Tg bioabbaubare Polyesterblöcke sind.

7. Der medizinische Gegenstand nach Anspruch 5, wobei der Polymerblock mit niedrigem Tg gewählt ist unter Homopolymer- und Copolymerblöcken, welche ein Monomer umfassen, das gewählt ist unter epsilon-Caprolacton, 3-Hydroxybutyrate, 3-Hydroxyvalerate, und Mischungen davon, und wobei der Polymerblock mit hohem T_{g} gewählt ist unter Homopolymer- und Copolymerblöcken, welche ein Monomer umfassen, das gewählt ist unter 1-Lactid, d-Lactid, Glycolid, und Mischungen davon.

## Revendications

1. Article médical comprenant une région polymère qui comprend un copolymère séquencé biodégradable comprenant un premier bloc de polymère biodégradable et un deuxième bloc de polymère biodégradable qui diffère du premier bloc de polymère biodégradable, ladite région polymère comprenant un premier domaine de phase formé dudit premier bloc de polymère et un deuxième domaine de phase formé dudit deuxième bloc de polymère, dans lequel au moins un des premier et deuxième domaines de phase est un domaine de phase discontinu sous la forme d'éléments en phase dispersée, dont sensiblement tous ont une longueur qui est inférieure à 1 micron, et
dans lequel ledit premier bloc de polymère biodégradable est un bloc de polymère à Tg faible et ledit deuxième bloc de polymère biodégradable est un bloc de polymère à Tg élevé.

2. Article médical de la revendication 1, **caractérisé en ce que** ledit article médical est un dispositif médical implantable ou insérable.

3. Article médical de la revendication 2, **caractérisé en ce que** ledit dispositif médical est un dispositif médical vasculaire choisi parmi une endoprothèse, une greffe, une endoprothèse vasculaire, un filtre de veine cave, une spire d'embolisation, une valve cardiaque, un dispositif d'accès ventriculaire gauche, un coeur artificiel et un dispositif de fermeture vasculaire.

4. Article médical de la revendication 1, dans lequel lesdits éléments en phase dispersée comprennent des éléments sphériques.

5. Article médical de la revendication 1, dans lequel ledit copolymère séquencé est un copolymère tribloc comprenant un bloc central à Tg faible et des blocs terminaux à Tg élevé.

6. Article médical de la revendication 5, dans lequel lesdits blocs de polymère à Tg faible et élevé sont des blocs de polyester biodégradables.

7. Article médical de la revendication 5, dans lequel ledit bloc de polymère à Tg faible est choisi parmi un homopolymère et des copolymères séquencés qui comprennent un monomère choisi parmi l'epsilon-caprolactone, le 3-hydroxybutyrate, le 3-hydroxyvalérate, et des combinaisons de ceux-ci, et dans lequel ledit bloc à Tg élevé est choisi parmi un homopolymère et des copolymères séquencés qui comprennent un monomère choisi parmi le 1-lactide, le d-lactide, le glycolide et des combinaisons de ceux-ci.
